# DEMANDE DE BREVET EUROPEEN

(11) **EP 1 518 542 A1**
(43) Date de publication de la demande: **30.03.2005**
(21) Numéro de dépôt: 04292193.2
(22) Date de dépôt: 13.09.2004
(51) Int. Cl.: A61K 7/13

(54) **Composition tinctoriale comprenant au moins une base paraphénylènediamine secondaire hydroxyalkylée, un coupleur et au moins un colorant direct cationique particulier**

(30) Priorité: 29.09.2003 FR 0311370
(71) Demandeur: L'OREAL, 75008 Paris (FR)
(72) Inventeur: Audousset, Marie-Pascale, 92600 Asnieres (FR)
(74) Mandataire: Fevrier, Murielle

(57) **Abrégé**

L'invention a pour objet une composition tinctoriale comprenant au moins une base d'oxydation para-phénylènediamine secondaire hydroxyalkylée, un coupleur et un colorant direct cationique dont la charge cationique est portée par un hétérocycle. L'invention a aussi pour objet l'utilisation de cette composition pour la teinture des fibres kératiniques, en particulier les fibres kératiniques humaines telles que les cheveux ainsi que le procédé de teinture mettant en oeuvre cette composition.

Une telle composition permet d'obtenir des couleurs variées résistantes.

## Description

L'invention a pour objet une composition tinctoriale comprenant au moins une base para-phénylènediamine secondaire hydroxyalkylée, un coupleur et un colorant direct cationique particulier. L'invention a aussi pour objet l'utilisation de cette composition pour la teinture des fibres kératiniques, en particulier les fibres kératiniques humaines telles que les cheveux ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, appelés généralement bases d'oxydation, tels que des ortho ou para-phénylènediamines, des ortho ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est déjà connu dans la demande de brevet DE10163251 d'utiliser des compositions tinctoriales comprenant une base d'oxydation para-phénylènediamine secondaire hydroxyalkylée pour la coloration des fibres kératiniques. Cependant, de telles bases ne permettent pas d'obtenir des couleurs de bonne qualité.

Le but de la présente invention est de fournir de nouvelles compositions tinctoriales, en particulier pour la teinture des fibres kératiniques qui permettent d'obtenir des nuances variées et de bonne qualité. En particulier, le but de l'invention est d'obtenir des colorations résistantes aux agents extérieurs tels que la sueur, les shampoings, la lumière, etc.

Ce but est atteint avec la présente invention qui a pour objet une composition tinctoriale comprenant, dans un milieu de teinture approprié,
- au moins une base d'oxydation de formule (1) ou les sels d'addition correspondants : 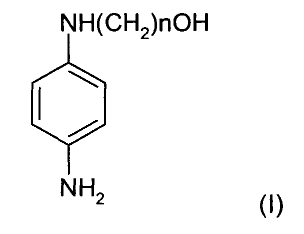
dans laquelle n est un nombre entier variant de 4 à 6,
- au moins un coupleur, et
- au moins un colorant direct cationique dont la charge cationique est portée par un hétérocycle.

L'invention a aussi pour objet un procédé de teinture mettant en oeuvre cette composition.

Un autre objet de l'invention est l'utilisation de la composition de la présente invention pour la teinture des fibres kératiniques, en particulier les fibres kératiniques humaines telles que les cheveux.

Enfin la présente invention a pour objet un dispositif à plusieurs compartiments destiné à la teinture des fibres kératiniques.

Dans la formule (I), les valeurs de n peuvent être 4, 5 ou 6, ce qui correspond respectivement aux composés 1-[N-(4'-hydroxybutyl)amino] 4-amino benzène, 1-[N-(5'-hydroxypentyl)amino] 4-amino benzène, 1-[N-(6'-hydroxyhexyl)amino] 4-amino benzène. Selon un mode de réalisation préféré, n est égal à 6.

La ou les bases d'oxydation de formule (1) sont présentes dans la composition de l'invention en général chacune en quantité comprise entre 0,001 et 10 % en poids environ du poids total de la composition tinctoriale, de préférence entre 0,005 et 6 %.

Le ou les coupleurs utiles dans la composition de la présente invention sont les coupleurs conventionnellement utilisés dans le domaine de la coloration des fibres kératiniques. Un tel coupleur est en général choisi parmi les méta-phénylènediamines, les méta-aminophénols, les méta-diphénols, les coupleurs naphtaléniques, les coupleurs hétérocycliques et leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

Le ou les colorants directs cationiques de la présente invention sont généralement choisis parmi les colorants xanthèniques, azoïques, azométhiniques et méthiniques porteurs d'au moins une charge cationique sur un hétérocycle.

De préférence, on utilisera des colorants azoïques, méthiniques ou azométhiniques porteurs d'au moins une charge cationique sur un hétérocycle.

Selon l'invention, le ou les colorants directs cationiques sont choisis parmi les colorants suivants :
- les colorants xanthéniques cationiques,
- les colorants directs azoïques ou azométhiniques cationiques, parmi lesquels on peut utiliser de préférence le Basic Blue 41, le Basic Blue 67, le Basic Red 22, le Basic Red 46,
- les colorants directs méthiniques cationiques, comme notamment le Basic Red 14, le Basic Yellow 13,
   ainsi que les colorants décrits dans la demande de brevet EP 1 025 834 suivants :
   - de formule (II)

      G-N=N-J (II)

      dans laquelle
      le symbole G représente un groupement choisi parmi les structures G₁ à G₃ suivantes : 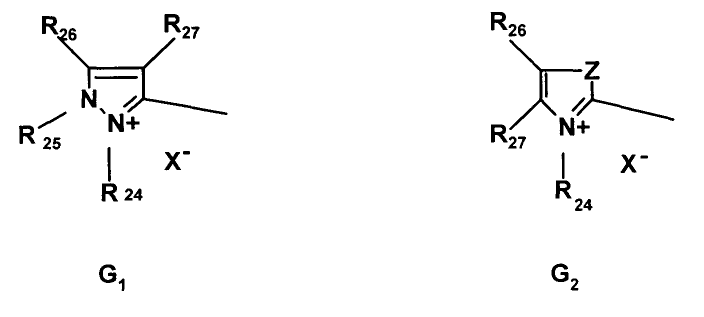 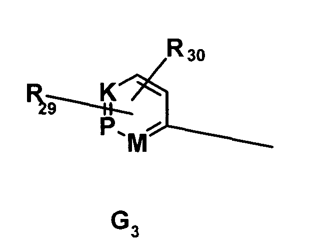 dans lesquelles,
      R₂₄ désigne un radical alkyle en C₁-C₄, un radical phényle pouvant être substitué par un radical alkyle en C₁-C₄ ou un atome d'halogène choisi parmi le chlore, le brome, l'iode et le fluor ;
      R₂₅ désigne un radical alkyle en C₁-C₄ ou un radical phényle;
      R₂₆ et R₂₇, identiques ou différents, représentent un radical alkyle en C₁-C₄ , un radical phényle, ou forment ensemble dans G₁ un cycle benzénique substitué par un ou plusieurs radicaux alkyle en C₁-C₄ , alcoxy en C₁-C₄, ou NO₂, ou forment ensemble dans G₂ un cycle benzénique éventuellement substitué par un ou plusieurs radicaux alkyle en C₁-C₄, alcoxy en C₁-C₄, ou NO₂;
      R₂₆ peut désigner en outre un atome d'hydrogène;
      Z désigne un atome d'oxygène, de soufre ou un groupement -NR₂₅;
      M représente un groupement -CH, -CR (R désignant alkyle en C₁-C₄), ou -NR₂₈(X⁻)ᵣ;
      K représente un groupement -CH, -CR (R désignant alkyle en C₁-C₄),ou -NR₂₈(X⁻)ᵣ;
      P représente un groupement -CH, -CR (R désignant alkyle en C₁-C₄),ou -NR₂₈(X⁻)ᵣ;
      r désigne zéro ou 1;
      R₂₈ représente un atome O⁻ , un radical alcoxy en C₁-C₄, ou un radical alkyle en C₁-C₄;
      R₂₉ et R₃₀, identiques ou différents, représentent un atome d'hydrogène ou d'halogène choisi parmi le chlore, le brome, l'iode et le fluor, un radical alkyle en C₁-C₄, alcoxy en C₁-C₄, un radical -NO₂;
      X⁻ représente un anion de préférence choisi parmi le chlorure, l'iodure, le méthyl sulfate, l'éthyl sulfate, l'acétate et le perchlorate;
      le symbole J représente :
      - (a) un groupement de structure J₁ suivante : 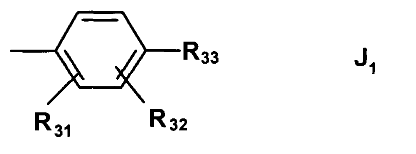 dans laquelle,
         R₃₁ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₃₄, -NR₃₅R₃₆, -NHCOalkyle en C₁-C₄, ou forme avec R₃₂ un cycle à 5 ou 6 chaînons contenant ou non un ou plusieurs hétéroatomes choisis parmi l'azote, l'oxygène ou le soufre;
         R₃₂ représente un atome d'hydrogène, un atome d'halogène choisi parmi le chlore, le brome, l'iode et le fluor, un radical alkyle en C₁-C₄, alcoxy en C₁-C₄,
         ou forme avec R₃₃ ou R₃₄ un cycle à 5 ou 6 chaînons contenant ou non un ou plusieurs hétéroatomes choisis parmi l'azote, l'oxygène ou le soufre;
         R₃₃ représente un atome d'hydrogène, un radical -OH, un radical -NHR₃₄, un radical -NR₃₅R₃₆;
         R₃₄ représente un atome d'hydrogène, un radical alkyle en C₁-C₄, un radical monohydroxyalkyle en C₁-C₄, polyhydroxyalkyle en C₂-C₄, un radical phényle;
         R₃₅ et R₃₆, identiques ou différents, représentent un radical alkyle en C₁-C₄ , un radical monohydroxyalkyle en C₁-C₄, polyhydroxyalkyle en C₂-C₄;
      - (b) un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle, et notamment un groupement de structure J₂ suivante : 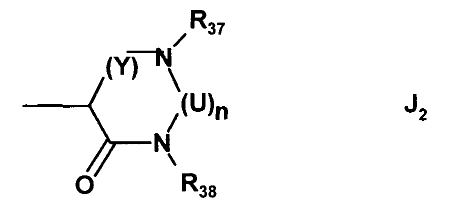 dans laquelle,
         R₃₇ et R₃₈, identiques ou différents, représentent un atome d'hydrogène , un radical alkyle en C₁₃-C₁₀ , un radical phényle;
         Y désigne le radical -CO- ou le radical -C(CH₃)= ;
         n représente 0 ou 1, avec, lorsque n désigne 1, U désigne le radical -CO-.
   - de formule (III) suivante : 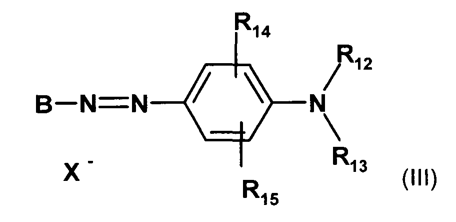 dans laquelle :
      R₁₂ représente un atome d'hydrogène ou un radical alkyle en C₁-C₄,
      R₁₃ représente un atome d'hydrogène, un radical alkyle pouvant être substitué par un radical -CN ou par un groupement amino, un radical 4'-aminophényle ou forme avec R₁₂ un hétérocycle éventuellement oxygéné et/ou azoté pouvant être substitué par un radical alkyle en C₁-C₄,
      R₁₄ et R₁₅, identiques ou différents, représentent un atome d'hydrogène, un atome d'halogène tel que le brome, le chlore, l'iode ou le fluor, un radical alkyle en C₁-C₄ou alcoxy en C₁-C₄, un radical -CN,
      X⁻ représente un anion de préférence choisi parmi le chlorure, le méthyl sulfate et l'acétate,
      B représente un groupement choisi par les structures B1 à B6 suivantes : 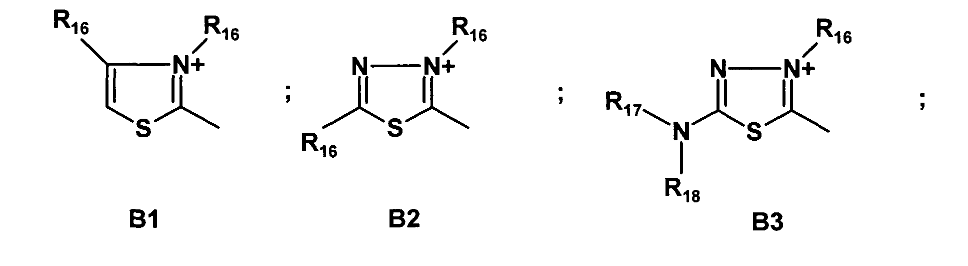 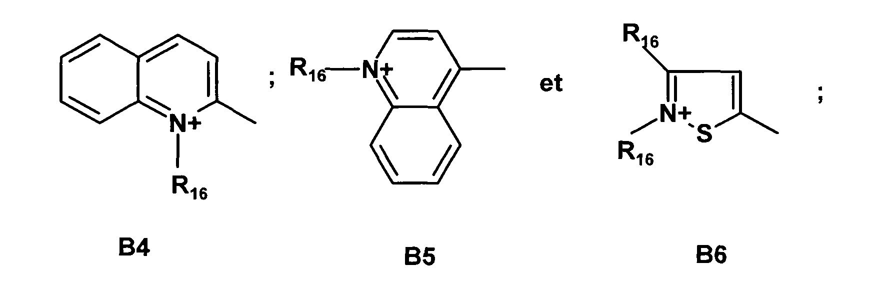
      dans lesquelles R₁₆ représente un radical alkyle en C₁-C₄,
      R₁₇ et R₁₈, identiques ou différents, représentent un atome d'hydrogène ou un radical alkyle en C₁-C₄,
   - les composés de formules (IV) et (V) suivantes : 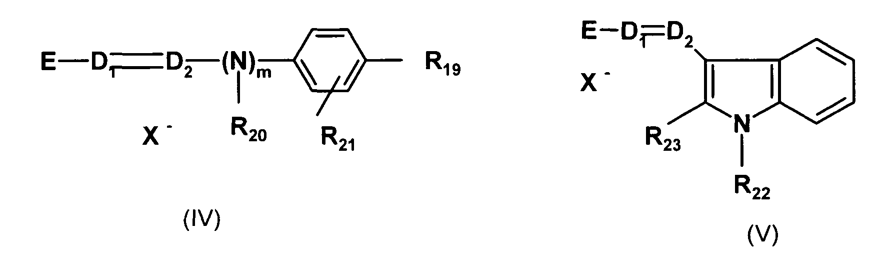 dans lesquelles :
      R₁₉ représente un atome d'hydrogène, un radical alcoxy en C₁-C₄, un atome d'halogène tel que le brome, le chlore, l'iode ou le fluor ou un radical amino,
      R₂₀ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
      R₂₁ représente un atome d'hydrogène ou d'halogène tel que le brome, le chlore, l'iode ou le fluor,
      R₂₂ et R₂₃, identiques ou différents, représentent un atome d'hydrogène ou un radical alkyle en C₁-C₄,
      D₁ et D₂, identiques ou différents, représentent un atome d'azote ou le groupement -CH,
      m représente 0 ou 1,
      étant entendu que lorsque R₁₉ représente un groupement amino non substitué, alors D₁ et D₂ représentent simultanément un groupement -CH et m = 0,
      X⁻ représente un anion de préférence choisi parmi le chlorure, le méthyl sulfate et l'acétate,
      E représente un groupement choisi par les structures E1 à E8 suivantes : 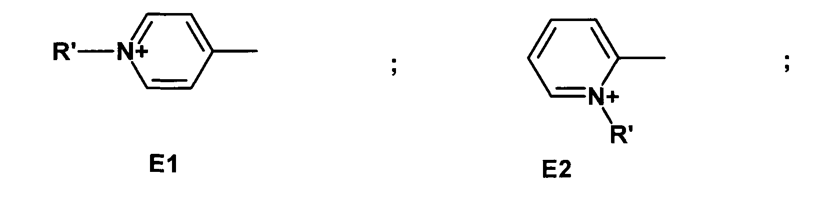 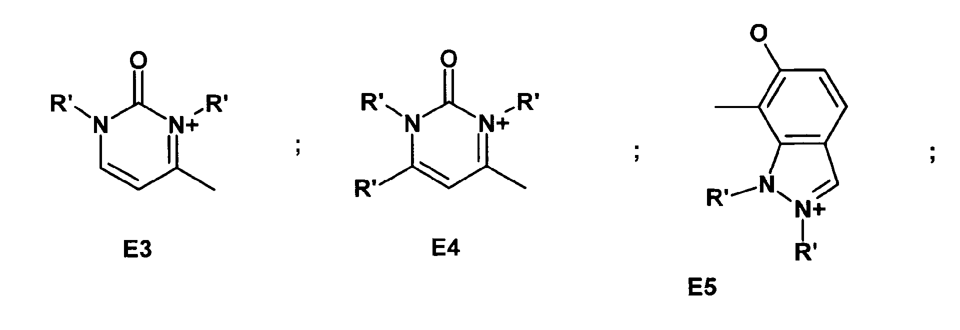 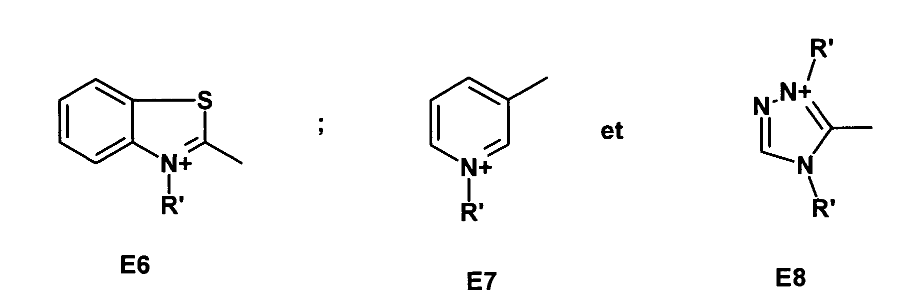 dans lesquelles R' représente un radical alkyle en C₁-C₄ ;
         lorsque m représente 0 et que D₁ représente un atome d'azote, alors E peut également désigner un groupement de structure E9 suivante : 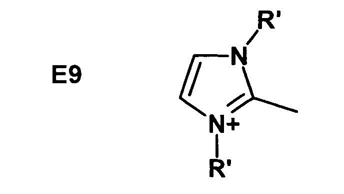 dans laquelle R' représente un radical alkyle en C₁-C₄.
   - les composés de formule (VI) suivante : 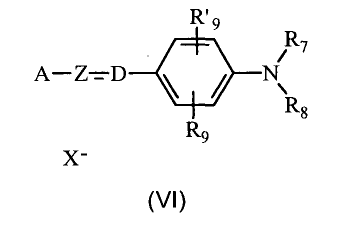 dans laquelle :
      Z et D représentent, identiques ou différents, un atome d'azote ou le groupement -CH,
      R₇ et R₈, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
      R₉ et R'₉, identiques ou différents, représentent un atome d'hydrogène ou d'halogène choisi parmi le chlore, le brome, l'iode et le fluor, un radical cyano, alkyl en C₁-C_{4,} alcoxy en C₁-C₄ ou acétyloxy,
      X⁻ représente un anion de préférence choisi parmi le chlorure, le méthyl sulfate et l'acétate,
      A représente un groupement choisi par les structures A1 à A18 suivantes : 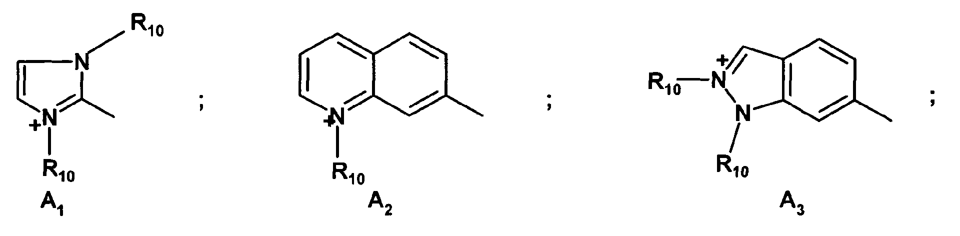 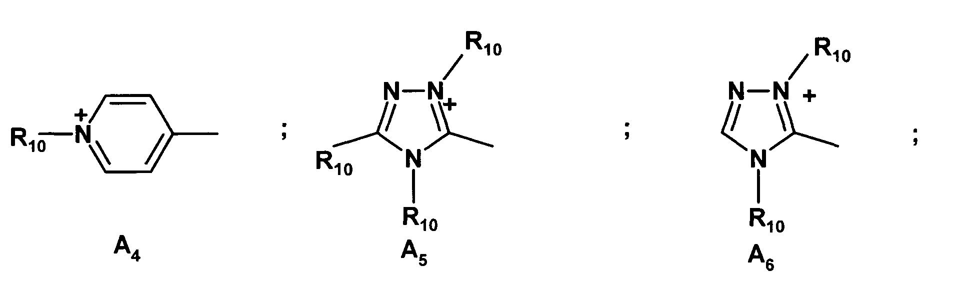 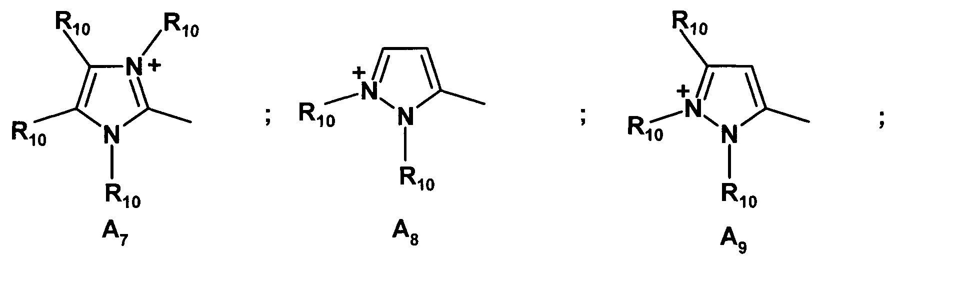 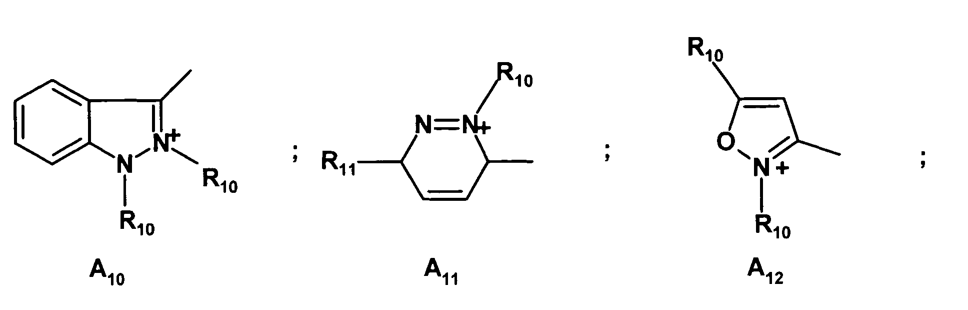 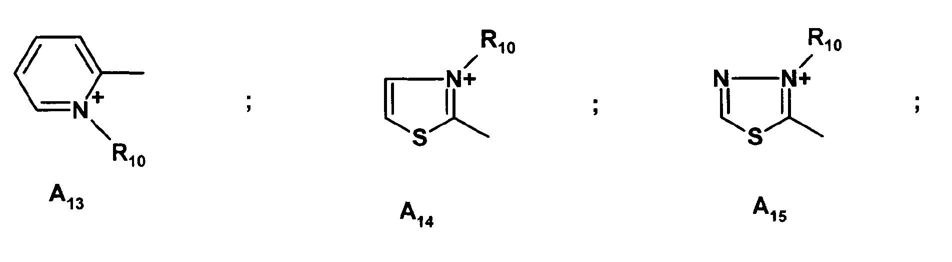 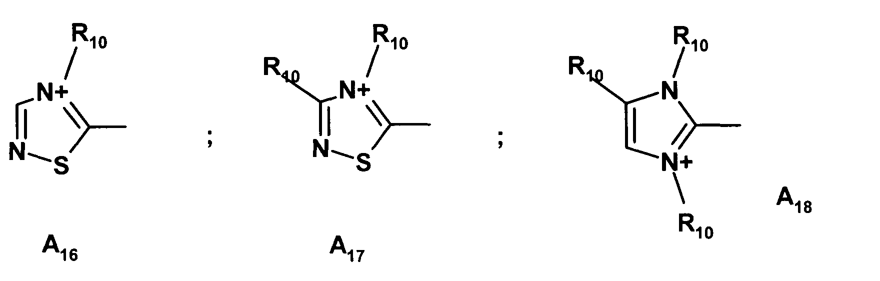
   dans lesquelles R₁₀ représente un radical alkyle en C₁-C₄ pouvant être substitué par un radical hydroxyle et R₁₁ représente un radical alcoxy en C₁-C₄,
- les colorants décrits dans la demande de brevet EP 714954 de formules : 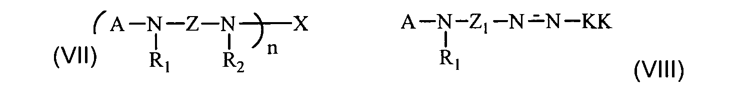 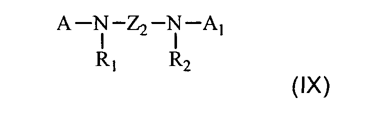
dans lesquelles
A et A₁, indépendamment l'un de l'autre, sont les restes de formule : 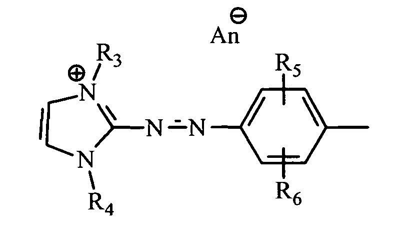 Z représente un reste de diamine aliphatique ou aromatique,
R₁ et R₂, indépendamment l'un de l'autre, représentent un atome d'hydrogène, un groupe alkyle en C₁-C₄ ou peuvent former ensemble avec 2 atomes d'azote auxquels ils sont rattachés ou avec Z et Z₂ un cycle à 5, 6 ou 7 chaînons,
X représente le reste d'un chaînon formant un pont,
n représente un nombre entier 2, 3 ou 4,
Z₁ représente un reste de diamines aromatique,
Z₂ représente un reste de diamines aliphatique,
KK représente un reste de composé coupleur,
R₃ et R₄, indépendamment l'un de l'autre, représentent un atome d'hydrogène ou un groupe alkyle en C₁-C₄,
R₅ et R₆, indépendamment l'un de l'autre, représentent un atome d'hydrogène, un groupe alkyle en C₁-C₄ ou un groupe alkoxy en C₁-C₄,
An représente un anion,
ainsi que les colorants cationiques décrits dans les demandes de brevet WO 95/01 772, WO 95/15 144, EP 714 954, EP 1 170 000 EP 1 166 753, EP 1 166 754 et EP 1 170 001, différents des colorants ci-dessus. On pourra se référer au passage de ces demandes consacré aux colorants cationiques.

De manière préférée, on utilisera les colorants Basic Red 51 de formule (X): 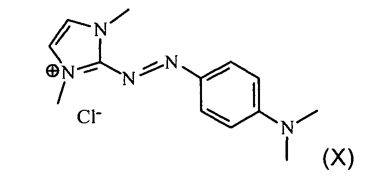 le Basic Yellow 87 de formule (XI) : 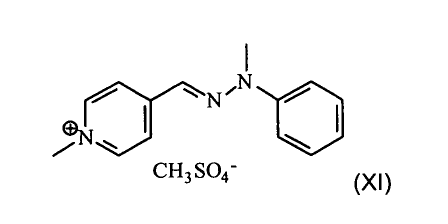 ainsi que le Basic Orange 31 de formule (XII) :

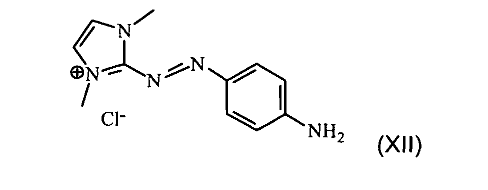

La composition de la présente invention peut en outre comprendre une ou plusieurs bases d'oxydation additionnelles classiquement utilisées en teinture d'oxydation. A titre d'exemple, ces bases d'oxydation additionnelles sont choisies parmi les para-phénylènediamines autres que celles de formule (I), les bis-phénylalkylènediamines, les para-aminophénols, les bis-para-aminophénols, les ortho-aminophénols, les bases hétérocycliques et leurs sels d'addition.

Parmi les para-phénylènediamines, on peut citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la N,N-diéthyl 3-méthyl para-phénylènediamine, la N,N-bis-(β-hydroxyéthyl) para-phénylènediamine, la N,N-bis-(β-hydroxyéthyl) 2-méthyl para-phénylènediamine, la N,N-bis-(β-hydroxyéthyl) 2-chloro para-phénylènediamine, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylène-diamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthytoxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols et les bis-para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino 2-chloro phénol, le 4-amino 2,6-dichloro phénol, le 4-amino 6-[(5'-amino 2'-hydroxy 3'-méthyl phényl)méthyl] 2-méthyl phénol, le bis-(5'-amino 2'-hydroxy phényl) méthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1 ,5-a]pyridin-5-yl)-(2-hydroxyéthyl)-amino]-éthanol ; le 2-[(3-amino-pyrazolo(1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation présentes dans la composition de l'invention sont en général présentes chacune en quantité comprise entre 0,001 et 10 % en poids environ du poids total de la composition tinctoriale, de préférence entre 0,005 et 6 %.

D'une manière générale, les sels d'addition des bases d'oxydation et des coupleurs utiles dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs conventionnels autres que ceux qui sont définis précédemment. Ces colorants peuvent être notamment choisis parmi les colorants nitrés de la série benzénique, les colorants directs azoïques, les colorants directs méthiniques.

Le milieu approprié pour la teinture appelé aussi support de teinture est généralement un support cosmétiquement acceptable constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers additifs utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les additifs ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (XIII) suivante : 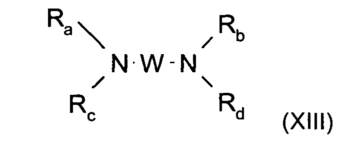 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, en présence d'un agent oxydant pendant un temps suffisant pour développer la coloration désirée. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Cette composition prête à l'emploi est ensuite appliquée sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus à l'exception de l'agent oxydant et un deuxième compartiment renferme un agent oxydant. Ce dispositif peut être équipé d'un moyen permettant de délivrer sur les cheveux le mélange souhaité, tel que les dispositifs décrits dans le brevet FR-2 586 913 au nom de la demanderesse.

Les exemples qui suivent servent à illustrer l'invention sans toutefois présenter un caractère limitatif.

### EXEMPLES

### EXEMPLES DE TEINTURE

On a préparé les compositions tinctoriales suivantes:

| | **Composition 1** | **Composition 2** | **Composition 3** |
|---|---|---|---|
| **1-[N-(6'- hydroxyhexyl)amino] 4-amino benzène; 2 HCl** (mole) | 0,005 | - | 0,005 |
| **1-[N-(5'- hydroxypentyl)amino] 4-amino benzène ; 2 HCl** (mole) | - | 0,005 | - |
| **6-hydroxy benzomorpholine** (mole) | 0,005 | - | - |
| **Résorcine** (mole) | - | 0,005 | - |
| **1-méthyl 2-hydroxy 4-(β-hydroxyéthyl)amino benzène** (mole) | - | - | 0,005 |
| **Basic orange 31** (g) | - | 0,15 | - |
| **Basic red 51** (g) | - | - | 0,15 |
| **Basic yellow 87 (g)** | 0,15 | - | - |

Ces compositions colorantes sont introduites dans le support de teinture suivant :

| | |
|---|---|
| Alcool oléique polyglycérolé à 2 moles de glycérol | 4 g |
| Alcool oléique polyglycérolé à 4 moles de glycérol (78 % M.A.) | 5,69 g M.A. |
| Acide oléique | 3,0 g |
| Amine oléique 2 OE commercialisée sous la dénomination d'ETHOMEEN O12 par la société AKZO | 7 g |
| Laurylamino succinamate de diéthylaminopropyle, sel de sodium à 55% M.A. | 3 g M.A |
| Alcool oléique | 5 g |
| Diéthanolamide d'acide oléique | 12 g |
| Propylène glycol | 3,5 g |
| Alcool éthylique | 7,0 g |
| Dipropylène glycol | 0,5 g |
| Monométhyléther de propylène glycol | 9 g |
| Métabisulfite de sodium en solution aqueuse à 35% M.A. | 0,455 g M.A |
| Acétate d'ammonium | 0,8 g |
| Antioxydant, séquestrant | q.s. |
| Parfum, conservateur | q.s. |
| Ammoniaque à 20% de NH₃ | 10 g |
| Précurseurs de colorants et colorants directs | Voir tableau ci-dessus |
| Eau déminéralisée | q.s.p. 100 g |

### Mode d'application

Au moment de l'emploi, chaque composition est mélangée avec un poids égal d'eau oxygénée à 20 volumes (6 % en poids).

Le mélange obtenu est appliqué sur des mèches de cheveux gris à 90 % de blancs naturels (BN) à raison de 30 g pour 3 g de cheveux. Après 30 min de pose, les mèches sont rincées, lavées avec un shampooing standard, rincées à nouveau puis séchées.
La coloration capillaire est évaluée de manière visuelle.

| | **Hauteur de ton 90%BN** | **Reflet 90%BN** |
|---|---|---|
| **Composition 1** | Blond Foncé | Vert Chromatique |
| **Composition 2** | Blond Foncé | Irisé Acajou |
| **Composition 3** | Châtain Clair | Violine Chromatique |

## Revendications

1. Composition tinctoriale comprenant, dans un milieu de teinture approprié,
• au moins une base d'oxydation de formule (I) ou les sels d'addition correspondants : 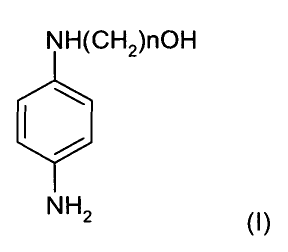
dans laquelle n est un nombre entier variant de 4 à 6,
• au moins un coupleur, et
• au moins un colorant direct cationique dont la charge cationique est portée par un hétérocycle.

2. Composition selon la revendication 1 dans laquelle n est égal à 6.

3. Composition selon la revendication 1 ou 2 dans laquelle le ou les coupleurs sont choisis parmi les méta-phénylènediamines, les méta-aminophénols, les méta-diphénols, les coupleurs naphtaléniques, les coupleurs hétérocycliques et leurs sels d'addition.

4. Composition selon l'une quelconque des revendications précédentes dans laquelle le colorant direct cationique est choisi parmi les colorants xanthèniques, azoïques, azométhiniques et méthiniques.

5. Composition selon la revendication 4 dans laquelle le colorant direct cationique est choisi parmi les colorants directs azoïques, azométhiniques ou méthiniques.

6. Composition selon l'une quelconque des revendications précédentes dans laquelle le colorant direct cationique est choisi parmi le groupe de colorants formé par : le Basic Blue 41, le Basic Blue 67, le Basic Red 22, le Basic Red 46, le Basic Red 14, le Basic Yellow 13 ainsi que les colorants suivants :
- les colorants de formule (II):
G - N = N - J (II)
dans laquelle
le symbole G représente un groupement choisi parmi les structures G₁ à G₃ suivantes : 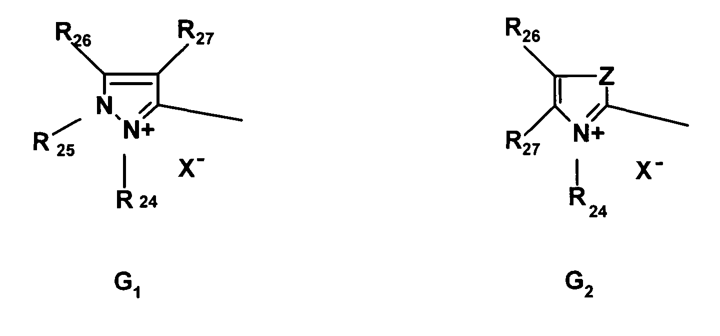 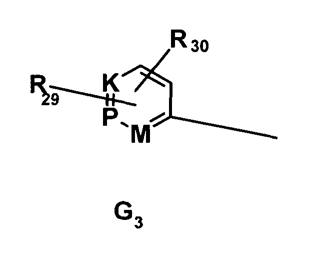 dans lesquelles,
R₂₄ désigne un radical alkyle en C₁-C₄, un radical phényle pouvant être substitué par un radical alkyle en C₁-C₄ ou un atome d'halogène choisi parmi le chlore, le brome, l'iode et le fluor ;
R₂₅ désigne un radical alkyle en C₁-C₄ ou un radical phényle;
R₂₆ et R₂₇, identiques ou différents, représentent un radical alkyle en C₁-C₄ , un radical phényle, ou forment ensemble dans G₁ un cycle benzénique substitué par un ou plusieurs radicaux alkyle en C₁-C₄ , alcoxy en C₁-C₄, ou NO₂, ou forment ensemble dans G₂ un cycle benzénique éventuellement substitué par un ou plusieurs radicaux alkyle en C₁-C₄ , alcoxy en C₁-C₄, ou NO₂;
R₂₆ peut désigner en outre un atome d'hydrogène;
Z désigne un atome d'oxygène, de soufre ou un groupement -NR₂₅;
M représente un groupement -CH, -CR (R désignant alkyle en C₁-C₄), ou -NR₂₈(X⁻)ᵣ;
K représente un groupement -CH, -CR (R désignant alkyle en C₁-C₄),ou -NR₂₈(X⁻)ᵣ; P représente un groupement -CH, -CR (R désignant alkyle en C₁-C₄),ou -NR₂₈(X⁻)ᵣ; r désigne zéro ou 1;
R₂₈ représente un atome O⁻ , un radical alcoxy en C₁-C₄, ou un radical alkyle en C₁-C_{4;}
R₂₉ et R₃₀, identiques ou différents, représentent un atome d'hydrogène ou d'halogène choisi parmi le chlore, le brome, l'iode et le fluor, un radical alkyle en C₁-C₄, alcoxy en C₁-C₄, un radical -NO₂;
X⁻ représente un anion de préférence choisi parmi le chlorure, l'iodure, le méthyl sulfate, l'éthyl sulfate, l'acétate et le perchlorate;
le symbole J représente :
- (a) un groupement de structure J₁ suivante : 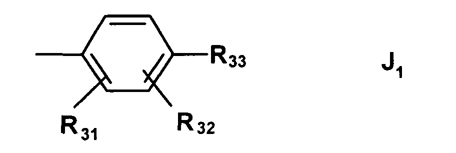 dans laquelle,
R₃₁ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₃₄, -NR₃₅R₃₆, -NHCOalkyle en C₁-C₄, ou forme avec R₃₂ un cycle à 5 ou 6 chaînons contenant ou non un ou plusieurs hétéroatomes choisis parmi l'azote, l'oxygène ou le soufre;
R₃₂ représente un atome d'hydrogène, un atome d'halogène choisi parmi le chlore, le brome, l'iode et le fluor, un radical alkyle en C₁-C₄, alcoxy en C₁-C₄,
ou forme avec R₃₃ ou R₃₄ un cycle à 5 ou 6 chaînons contenant ou non un ou plusieurs hétéroatomes choisis parmi l'azote, l'oxygène ou le soufre;
R₃₃ représente un atome d'hydrogène, un radical -OH, un radical -NHR₃₄, un radical -NR₃₅R₃₆;
R₃₄ représente un atome d'hydrogène, un radical alkyle en C₁-C₄, un radical monohydroxyalkyle en C₁-C₄, polyhydroxyalkyle en C₂-C₄, un radical phényle;
R₃₅ et R₃₆, identiques ou différents, représentent un radical alkyle en C₁-C₄ , un radical monohydroxyalkyle en C₁-C₄, polyhydroxyalkyle en C₂-C₄;
- (b) un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle,
et notamment un groupement de structure J₂ suivante : 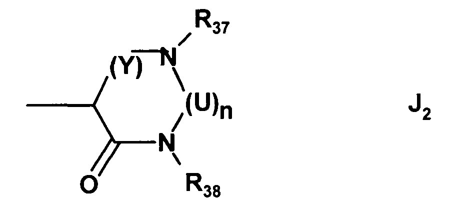
dans laquelle,
R₃₇ et R₃₈, identiques ou différents, représentent un atome d'hydrogène , un radical alkyle en C₁₃-C₁₀ , un radical phényle;
Y désigne le radical -CO- ou le radical -C(CH₃)= ;
n représente 0 ou 1, avec, lorsque n désigne 1, U désigne le radical -CO- ;
- les composés de formule (III) suivante : 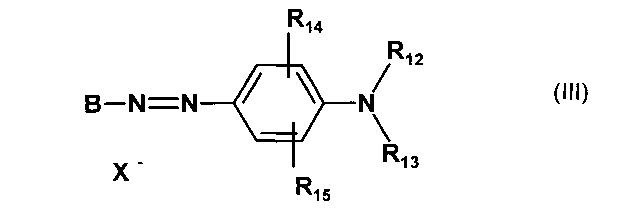 dans laquelle :
R₁₂ représente un atome d'hydrogène ou un radical alkyle en C₁-C₄,
R₁₃ représente un atome d'hydrogène, un radical alkyle pouvant être substitué par un radical -CN ou par un groupement amino, un radical 4'-aminophényle ou forme avec R₁₂ un hétérocycle éventuellement oxygéné et/ou azoté pouvant être substitué par un radical alkyle en C₁-C₄,
R₁₄ et R₁₅, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
X⁻ représente un anion de préférence choisi parmi le chlorure, le méthyl sulfate et l'acétate,
B représente un groupement choisi par les structures B1 à B6 suivantes : 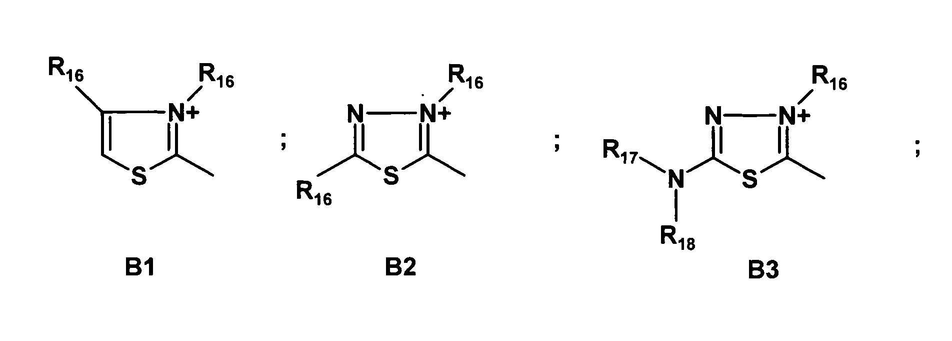 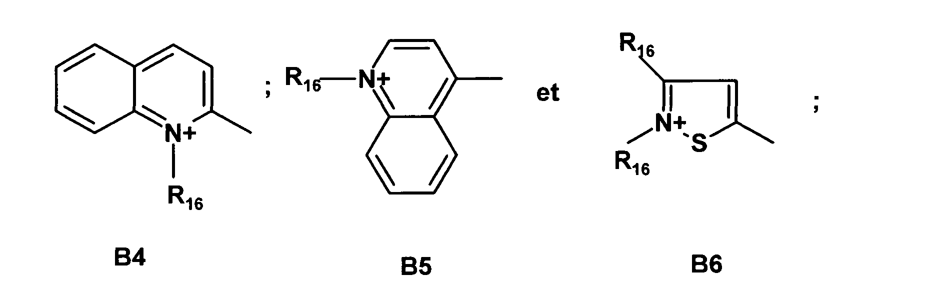
dans lesquelles R₁₆ représente un radical alkyle en C₁-C₄,
R₁₇ et R₁₈, identiques ou différents, représentent un atome d'hydrogène ou un radical alkyle en C₁-C₄,
- les composés de formules (IV) et (V) suivantes : 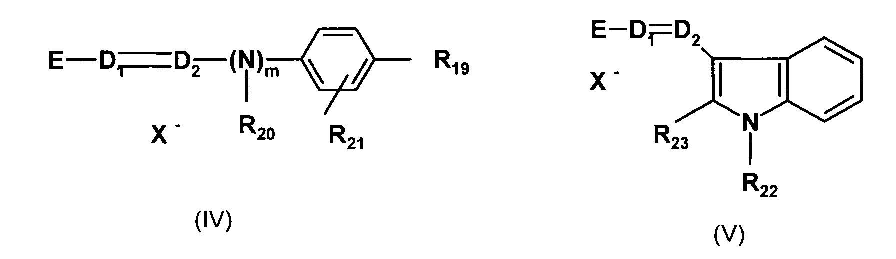 dans lesquelles :
R₁₉ représente un atome d'hydrogène, un radical alcoxy en C₁-C₄, un atome d'halogène tel que le brome, le chlore, l'iode ou le fluor ou un radical amino,
R₂₀ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
R₂₁ représente un atome d'hydrogène ou d'halogène tel que le brome, le chlore, l'iode ou le fluor,
R₂₂ et R₂₃, identiques ou différents, représentent un atome d'hydrogène ou un radical alkyle en C₁-C₄,
D₁ et D₂, identiques ou différents, représentent un atome d'azote ou le groupement -CH,
m représente 0 ou 1,
étant entendu que lorsque R₁₉ représente un groupement amino non substitué, alors D₁ et D₂ représentent simultanément un groupement -CH et m = 0,
X⁻ représente un anion de préférence choisi parmi le chlorure, le méthyl sulfate et l'acétate,
E représente un groupement choisi par les structures E1 à E8 suivantes : 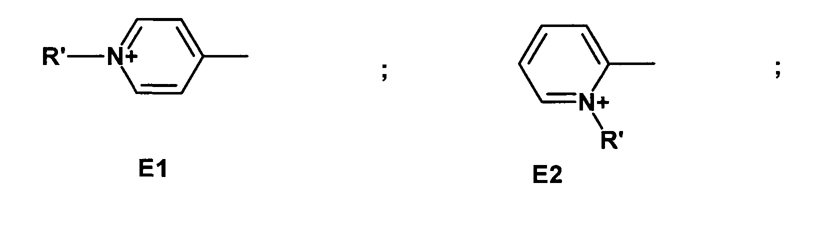 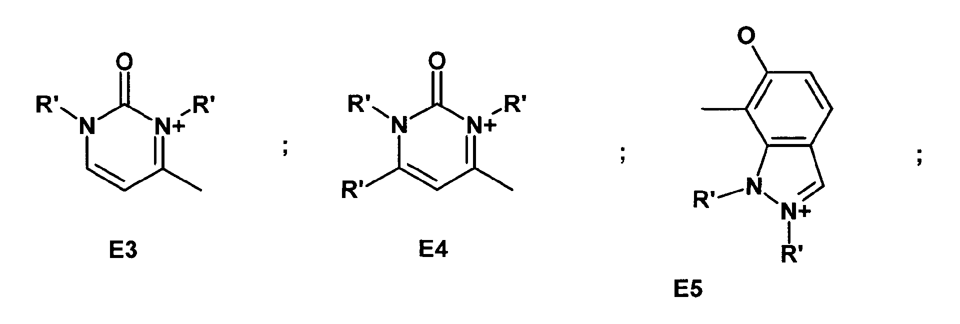 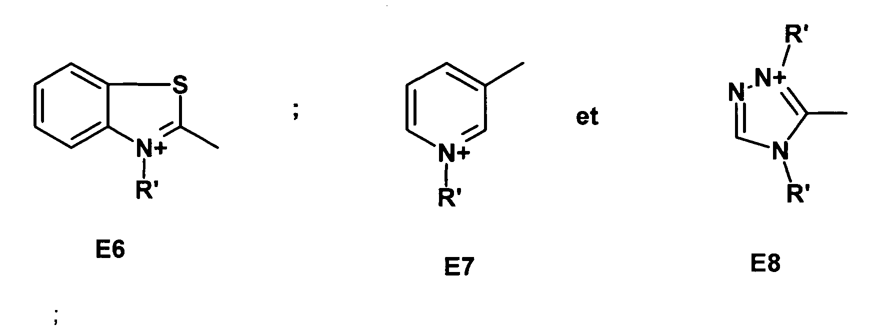 dans lesquelles R' représente un radical alkyle en C₁-C₄ ;
lorsque m représente 0 et que D₁ représente un atome d'azote, alors E peut également désigner un groupement de structure E9 suivante : 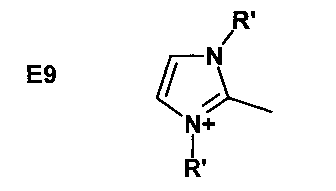 dans laquelle R' représente un radical alkyle en C₁-C₄.
- les colorants de formule (VI) 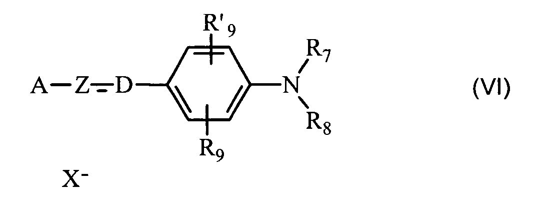
dans laquelle :
Z et D représentent, identiques ou différents, un atome d'azote ou le groupement -CH-,
R₇ et R₈, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₉ et R'₉, identiques ou différents, représentent un atome d'hydrogène ou d'halogène choisi parmi le chlore, le brome, l'iode et le fluor, un radical cyano, alkyl en C₁-C_{4,} alcoxy en C₁-C₄ ou acétyloxy,
X⁻ représente un anion de préférence choisi parmi le chlorure, le méthyl sulfate et l'acétate,
A représente un groupement choisi par les structures A1 à A18 suivantes : 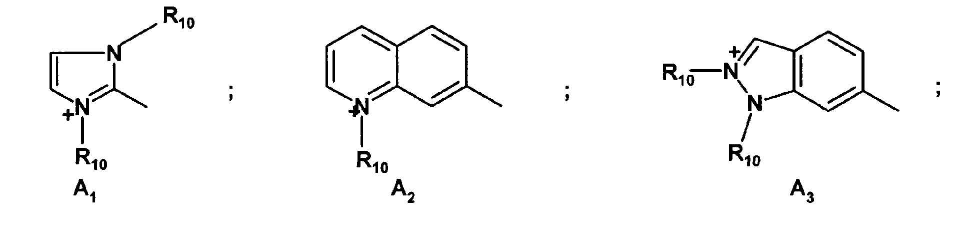 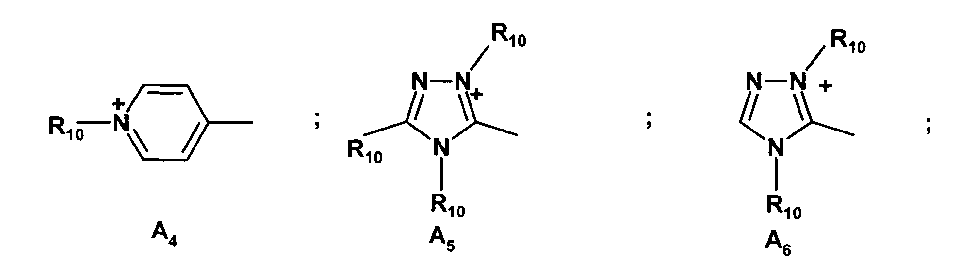 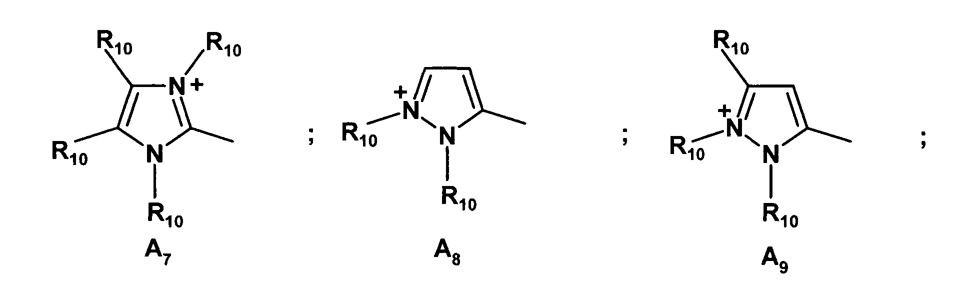 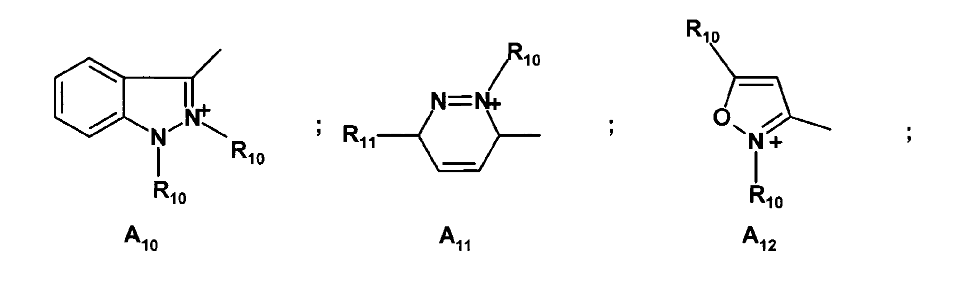 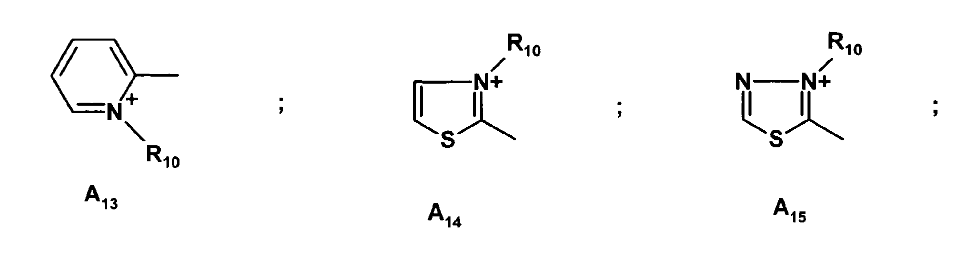 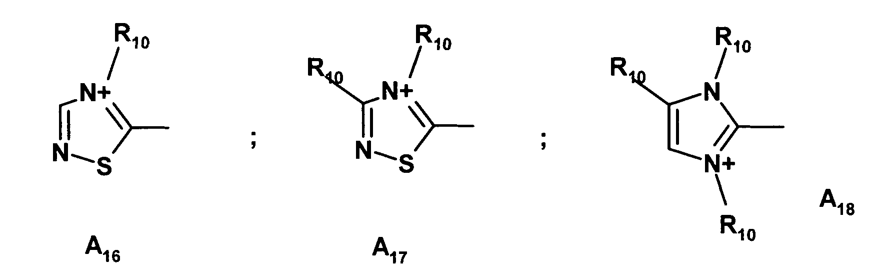 dans lesquelles R₁₀ représente un radical alkyle en C₁-C₄ pouvant être substitué par
un radical hydroxyle et R₁₁ représente un radical alcoxy en C₁-C₄,
- les composés de formules ( VII), (VIII) et (IX) : 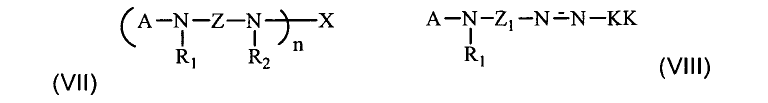 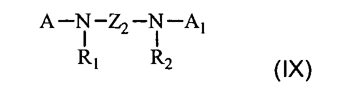
dans lesquelles
A et A₁, indépendamment l'un de l'autre, sont les restes de formule : 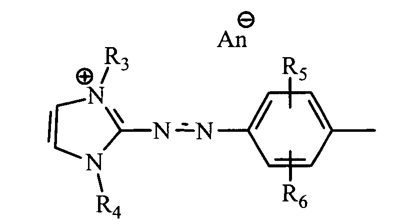 Z représente un reste de diamine aliphatique ou aromatique,
R₁ et R₂, indépendamment l'un de l'autre, représentent un atome d'hydrogène, un groupe alkyle en C₁-C₄ ou peuvent former ensemble avec 2 atomes d'azote auxquels ils sont rattachés ou avec Z et Z₂ un cycle à 5, 6 ou 7 chaînons,
X représente le reste d'un chaînon formant un pont,
n représente un nombre entier 2, 3 ou 4,
Z₁ représente un reste de diamines aromatique,
Z₂ représente un reste de diamines aliphatique,
KK représente un reste de composé coupleur,
R₃ et R₄, indépendamment l'un de l'autre, représentent un atome d'hydrogène ou un groupe alkyle en C₁-C₄,
R₅ et R₆, indépendamment l'un de l'autre, représentent un atome d'hydrogène, un groupe alkyle en C₁-C₄ ou un groupe alkoxy en C₁-C₄,
An représente un anion.

7. Composition selon l'une quelconque des revendications 1 à 5 **caractérisée par le fait que** le colorant direct cationique est choisi parmi le Basic Red 51, le Basic yellow 87 et le Basic Orange 31.

8. Composition selon l'une quelconque des revendications précédentes comprenant au moins une base d'oxydation additionnelle choisie parmi les para-phénylènediamines autres que celles de formule (I) telles que définies à la revendication 1 ou 2, les bis-phénylalkylènediamines, les para-aminophénols, les bis-para-aminophénols, les ortho-aminophénols, les bases hétérocycliques et leurs sels d'addition.

9. Composition selon l'une quelconque des revendications précédentes dans laquelle la quantité de chacun des coupleurs est comprise entre 0,001 et 10 % en poids environ du poids total de la composition tinctoriale.

10. Composition selon l'une quelconque des revendications précédentes dans laquelle la quantité de chacune des bases d'oxydation est comprise entre 0,001 et 10 % en poids environ du poids total de la composition tinctoriale.

11. Composition selon l'une quelconque des revendications précédentes comprenant un colorant direct autre que ceux qui sont définis à l'une quelconque des revendications 1 et 4 à 7 choisi parmi les colorants nitrés de la série benzénique, les colorants directs azoïques, les colorants directs méthiniques.

12. Composition selon l'une quelconque des revendications précédentes comprenant de plus un additif choisi parmi les agents tensio-actifs anioniques, cationiques, non-ioniques, amphotères, zwitterioniques ou leurs mélanges, les polymères anioniques, cationiques, non-ioniques, amphotères, zwitterioniques ou leurs mélanges, les agents épaississants minéraux ou organiques, les agents épaississants associatifs polymères anioniques, cationiques, non ioniques ou amphotères, les agents antioxydants, les agents de pénétration, les agents séquestrants, les parfums, les agents tampons, les agents dispersants, les agents de conditionnement, les agents filmogènes, les céramides, les agents conservateurs, les agents opacifiants.

13. Composition selon l'une quelconque des revendications précédentes comprenant un agent oxydant.

14. Procédé de teinture d'oxydation des fibres kératiniques, **caractérisé en ce qu'**on applique sur les fibres une composition tinctoriale telle que définie dans l'une quelconque des revendications 1 à 12 en présence d'un agent oxydant pendant un temps suffisant pour développer la coloration désirée.

15. Procédé selon la revendication 14 dans lequel l'agent oxydant est choisi parmi le peroxyde d'hydrogène, le peroxyde d'urée, les bromates de métaux alcalins, les persels, les peracides et les enzymes oxydases.

16. Dispositif à plusieurs compartiments dans lequel un premier compartiment contient une composition tinctoriale telle que définie à l'une quelconque des revendications 1 à 12 et un deuxième compartiment contient un agent oxydant.

17. Utilisation de la composition définie aux revendications 1 à 13 pour la teinture de fibres kératiniques.
